# EUROPEAN PATENT APPLICATION

(11) **EP 1 557 176 A1**
(43) Date of publication of application: **27.07.2005**
(21) Application number: 04001570.3
(22) Date of filing: 26.01.2004
(51) Int. Cl.: A61K 38/29, A61P 19/08

(54) **Treatment of bone metastases by means of PTH receptor agonists**

(71) Applicant: Ferring B.V., 2132 JX Hoofddorp (NL)
(72) Inventor: Rivière, Pierre J-M., San Diego CA 92130 (US); Akinsanya, Karen, Chula Vista CA 91913 (US)
(74) Representative: Poulsen, Niels Jakob

(57) **Abstract**

Symptoms associated with the growth of bone-metastasized cancer or bone-originated cancer, such as bone loss, bone fracturing, and pain, can be ameliorated by administering a effective amount of a PTH receptor agonist, which can be PTH, a PTH fragment, or an analog thereof or parathyroid hormone-related protein, a fragment or an analog thereof.

## Description

### Field of the Invention

The invention relates to parathyroid hormone analogs and parathyroid hormone-related protein analogs and their use in the preparation of medicaments for the treatment of complications from bone metastasizing cancers.

### Background of the Invention

Prostate and breast cancer are associated with a high prevalence of metastatic disease in bone. For example, of the approximately 180,000 women who are diagnosed annually with breast cancer in the US, 30% of them will develop metastases and 70% of these will involve bone. Other cancers characterized by metastatic bone disease include those from the lung, kidney and thyroid.

Bone metastases are commonly categorized as osteolytic or osteoblastic, with multiple different factors being responsible for each. Recent data suggests that osteolytic and osteoblastic lesions are the two extremes. Morphological analysis shows that most bone metastases have both osteoblastic and osteolytic lesions.

In metastatic disease a mixed pattern of bone destruction and bone formation is common. Altered bone remodeling in metastatic cancer is caused by over-activation of osteoblasts and/or osteoclasts and in some cases a combined effect of the cancer cells on bone resorption, which creates an imbalance in natural bone turnover. Apart from the deleterious effects of increased osteoclasts and/or osteoblast activity on bone density and the subsequent loss of integrity of bone resulting in fractures, or increased pressure and invasion in bone, the pain associated with bone metastases substantially erodes the patient's quality of life. Individuals with metastatic bone disease also have difficulty to stand or sit secondary to pain.

Similar complications with bone and underlying pathology also are present in primary bone cancers (cancers that arise in bone) such as myeloma, lymphoma, and primary mesenchymal bone tumors, such as chondrosarcoma, malignant fibrous histiocytoma, fibrosarcoma, osteosarcoma, sarcoma in Paget's disease, and sarcoma in irradiated bone.

Parathyroid hormone (PTH), parathyroid related-protein (PTHrP), and analogs thereof are among many agents that have been proposed for the treatment of osteoporosis, which is a disease characterized by a reduced amount of bone tissue and increased risk of fractures.

Parathyroid hormone (PTH) is an 84 amino acid polypeptide which is normally secreted from the parathyroid glands. PTH has an important physiological role to maintain serum calcium within a narrow range. PTH acts directly on bone and kidney and indirectly on the intestines. PTH production in healthy individuals is closely regulated by the concentration of serum ionized calcium. The increase in PTH levels increases the rate of bone resorption, thereby increasing the calcium flow from bone into blood, reduces the renal clearance of calcium, and increases the efficiency of calcium absorption in the intestines.

The physiological role of the parathyroid hormone-related protein (PTHrP), a 139 to 173 amino acid residues peptide, is not fully understood, but is thought to be acting principally as a paracrine or autocrine factor. PTHrP plays a role in fetal development as well as in adult physiology. In adults, PTHrP is thought to have little to do with calcium homeostasis, except in disease states.

PTH and PTHrP are distinct proteins and products of different genes. However, they share a similar bioactivity profile and a very limited sequence homology, indicating that they may have evolved from a common ancestral gene. Eight out of the 13 first amino acid residues at the N-terminus are identical. Both PTH and PTHrP bind to the PTH receptor (often referred to as the PTH/PTHrP receptor) and stimulate the same intracellular signaling pathways.

The mature circulating form of parathyroid hormone is comprised of 84 amino acid residues. For most bone-related activities, the truncated form of PTH, PTH(1-34), is a full agonist like the native 84 amino-acid hormone. Amino-terminal truncation results in polypeptides that are competitive antagonists of PTH-stimulated adenylate cyclase (Rosenblatt, *et al., Endocrinol.* (1980) 107: 545-50). In contrast, carboxyl-terminal truncations of PTH(1-34) produce agonists with progressively lower affinities. PTH(1-25) is reported to be essentially inactive (see Rosenblatt, M., *in* ENDOCRINOLOGY OF CALCIUM METABOLISM 103-42, Parsons, J.A. (ed.), Ravens Press (1982)). The principal receptor-binding domain of PTH is reported to include amino acid residues 25-34 and the principal activation domain is reported to include amino acid residues 1-6.

Under some circumstances, PTH is a bone anabolic agent and promotes bone formation. Yet PTH can stimulate bone resorption as well. PTH administered continuously causes an increase in the number of bone cells, including osteoclasts, and an increase in bone remodeling. These increases are apparent within hours after PTH administration and persist for hours after PTH is withdrawn. PTH administration intermittently over days in humans and animals leads to a net stimulation of bone formation. For example, *see* Neer, *et al., N. Engl. J. Med.* (2001) 344: 1434-41. In contrast, continuous exposure to elevated levels of PTH leads to osteoclast-mediated bone resorption. Use of PTH and PTHrP analogs as agents to treat osteoporosis is described *inter alia* in US 6,316,410.

Under the brand name Forteo® (Eli Lilly), PTH (1-34) in the form of teriparatide acetate has been approved in the U.S. for the treatment of osteoporosis. Studies in rats demonstrated that teriparatide acetate demonstrated an increased risk of osteosarcoma (a malignant bone tumor) that was dependent on dose and treatment duration. Accordingly, Forteo® is contraindicated for individuals who are at increased risk for osteosarcoma or who have bone cancer or other cancers that have metastasized to bone.

A need exists for agents that can be used to treat complications from bone metastases such as bone loss, bone fracturing and bone pain. Preferably, such agents will have improved functional activity with minimum adverse side effects.

### Summary of the Invention

The present inventors have discovered that parathyroid hormone receptor agonists ameliorate symptoms associated with bone loss, likelihood of fracture, and bone pain associated with growth of bone metastasized cancer or primary bone cancer. This discovery contravenes an accepted understanding in the field against the use ofparathyroid hormone, such as PTH(1-34), in individuals with a likelihood of bone cancer or metastatic disease.

Accordingly, the present invention provides a method of ameliorating symptoms associated with bone metastasized cancer or primary bone cancer by administering an effective amount of a PTH receptor agonist. In some embodiments, the PTH receptor agonist is PTH or a fragment or analog thereof, or parathyroid hormone-related protein (PTHrP) or a fragment or analog thereof. In a preferred embodiment, the PTH receptor agonist is PTH(1-34), most preferably teriparatide acetate.

In accordance with one embodiment, the treated individual has bone metastatic cancer. Bone metastatic cancer may be breast cancer, prostate cancer, lung cancer, kidney cancer, thyroid cancer or myeloma.

In accordance with another embodiment, the treated individual has bone-originated cancer. The bone-originated cancer is preferably a sarcoma.

In accordance with yet another embodiment, the PTH receptor agonist is administered to the patient daily, every second day, every third day, twice per week, every fourth day, every fifth day, every sixth day, or once per week.

In another aspect, PTH receptor agonists are used in the preparation of a medicament, particularly a medicament for the treatment of bone loss, likelihood of fracture, and bone pain associated with growth of bone metastasized cancer or primary bone cancer.

These and other embodiments of the invention are described in greater detail below.

### Brief Description of the Figures

FIG. 1 is a graph showing optical density readings from x-ray radiographs of PTH treated and untreated animals with metastatic cancer disease. Scans were integrated to quantify total bone density.
FIG. 2 shows pain evaluation in PTH treated and untreated animals with metastatic cancer disease. At day 14 following establishment of bone tumor metastasis, PTH( 1-34) ameloriated pain measured by limb use, limb guarding and spontaneous flinching. PTH (1-34) treatment did not effect tactile hypersensitivity (evoked pain).

### Detailed Description of the Invention

Throughout this description, the name of an amino acid may be followed by a superscript number that refers to the position of the amino acid in the sequence. For example, Glu²² means there is a glutamic acid at position 22. Polypeptide sequences mentioned herein are written according to the generally accepted convention wherein the N-terminal amino acid is on the left and the C-terminal amino acid is on the right. Numbering starts at the N-terminus and proceeds toward the C-terminus. Accordingly, [Leu^{23,28,31}]hPTHrP means a polypeptide having a sequence of hPTHrP in which the wild type residues Phe²³, Ile²⁸, and Ile³¹ have each been replaced with leucine.

"PTH receptor agonist" is a phrase employed here to denote both natural and non-natural molecules that bind and activate the PTH receptor. Within this definition are PTH peptides and peptide analogues and PTHrP peptides and analogues. PTH and PTHrP peptides and analogues include full length and fragment analogs which have agonist properties similar to PTH and PTHrP with respect to the PTH receptor. PTH peptides and peptide analogues and PTHrP peptides and analogues are by definition exclusive compositions.

PTHrP analogues that have PTH receptor-agonist activity include variants that are modified relative to the native molecule, by virtue, for instance, of one or more substitutions, deletions, insertions, inversions or cyclizations, but that display the biological activities of full length parathyroid hormone.

In this context, the term "biologically active" should be understood as eliciting a sufficient response in a bioassay for PTH activity, such as the rat osteosarcoma cell-based assay for PTH-stimulated adenylate cyclase production. See Rabbani, *et al*. (1988) *Endocrinol.* 123: 2709. Stability-enhanced variants of PTH are known, for example, in PCT application WO 93/20203, and constitute another useful form of PTH receptor agonist. Variants of PTH can incorporate, for example, amino acid substitutions that improve PTH stability and half-life, such as the replacement of methionine residues at positions 8 and/or 18, and replacement of asparagine at position 16. Cyclized PTH analogues are disclosed in PCT application WO 98/05683, *inter alia*.

The category of PTH receptor agonists encompasses naturally occurring human PTH(1-84), synthetic or recombinant PTH (rPTH), and fragments of PTH. The present invention comprehends the use of fragments of PTH in amounts giving equivalent biological activity to PTH(1-84), in the pharmaceutical formulations as described above. Similarly, PTH receptor agonists include naturally occurring PTHrP, synthetic or recombinant forms of this protein, and biologically active fragments thereof.

As used herein "hPTH(1-34)" refers to a shortened human sequence of PTH ("hPTH"), as set forth in SEQ ID NO:1. Similarly, "bPTH(1-34)" refers to the bovine sequence of PTH ("bPTH"), as set forth in SEQ ID NO:2. "hPTHrP(1-34)" refers to a shortened form of human parathyroid hormone related protein ("hPTHrP"), as set forth in SEQ ID NO:3. hPTH(1-34) is a preferred PTH receptor agonist.

The length of the PTH or PTHrP analog can be shortened relative to full-length PTH or PTHrP by from anywhere between 1 to 50 or more amino acids. Preferably the PTH or PTHrP analog contains from 30 to 40 amino acids. Most preferably it contains 34 amino acids.

The PTH or PTHrP analogs of the invention can be based on PTH or PTHrP sequences of any species, although it is preferred that human PTH or PTHrP or bovine PTH or PTHrP serve as the basis for the analog.

PTH receptor agonists also encompass analogs that have a modified carboxy terminus. Preferred modified analogs include amidated polypeptides, in which the terminal carboxy group (-COOH) is converted to a carboxamide group (-C(O)NH₂). Also alkylamide analogs are conceivable.

Therefore, "hPTH(1-34)NH₂," as used herein, refers to a truncated form of human parathyroid hormone having a carboxamide group at the carboxy terminus. "bPTH(1-34)NH₂," and "hPTHrP(1-34)NH₂," are analogously denoted.

As used herein, "Ψ(CH₂NH)" indicates a reduced peptide bond between two residues. For example, SEQ ID NO:4 has a reduced peptide bond between Ala¹ and Val². Table 1 contains a list of common amino acids abbreviations used herein. All chiral amino acid residues are of the S-configuration.

**Table 1:**

| Amino acid abbreviations | |
|---|---|
| Abbreviation | Amino Acid |
| Nle | norleucine |
| Aib | 2-aminoisobutyric acid |
| 2-Nal | 2-naphthylalanine |
| Thi | 2-thienylalanine |
| β-Ala | beta-alanine |
| β-hLeu | beta-homo-leucine |
| β-hAsp | beta-aspartic acid |
| β-hSer | beta-homo-serine |
| β-hGlu | beta-homo-glutamic acid |
| β-hThr | beta-homo-threonine |
| β-hArg | beta-homo-arginine |
| β-hLys | beta-homo-lysine |
| β-hDap | beta-homo-diaminopropionic acid (3,4-diaminobutyric acid) |
| β-hDbu | beta-homo-diaminobutyric acid (3,5-diaminopentanoic acid) |
| β-hOrn | beta-homo-ornithine |
| β-hAla | beta-homo-alanine |
| β-hAsn | beta-homo-asparagine |
| β-hGln | beta-homo-glutamine |

In some aspects of PTH receptor agonist, substitutions are made at positions 16-20 of PTH or PTHrP, preferably at positions 17-19. As examples of this, provided below in Table 2 are analogs of bPTH that are substituted at the indicated amino acids. The wild-type bPTH amino acid sequence, SEQ ID NO:2, is provided for comparison. Similar substitutions can be made to hPTH (SEQ ID NO:1) and to hPTHrP (SEQ ID NO:3), which sequences also are listed for comparison.

Thus β-amino acid-containing analogs can have the following structural permutations, alone or in combination, and optionally with further amino acid substitutions as described elsewhere herein:
Position 16: β-hAsn, β-hSer, β-hGln, β-hThr, β-hAla, β-Ala, β-hRaa, wherein
   β-hRaa = NH-CH(R)CH₂CO, where R=Et, nPr, (CH₂)ₙCONH₂ (n=0, 3 and 4).
Position 17: β-hAsp, β-hSer, β-hGlu, β-hThr, β-hAla, β-Ala, β-hRaa, wherein
   β-hRaa = NH-CH(R)CH₂CO, where R=Et, nPr, (CH₂)ₙCO₂H (n=0, 3 and 4).
Position 18: β-hMet, β-hNle, β-hLeu, β-hIle, β-hVal, β-hAla, β-Ala, β-hRaa, wherein
   β-hRaa = NH-CH(R)CH₂CO, where R=Et, nPr.
Position 19: β-hAsp, β-hSer, β-hGlu, β-hThr, β-hArg, β-hLys, β-hDap, β-hDbu, β-hOrn, β-hAla,
   β-Ala, β-hRaa, wherein β-hRaa = NH-CH(R)CH₂CO, where R=(CH₂)ₙGuanidino
   (n=1, 2 and 4), (CH₂)ₙCO₂H (n=0, 3 and 4).
Position 20: β-hArg, β-hLys, β-hDap, β-hOrn, β-hAla, β-Ala, β-hRaa, wherein
   β-hRaa = NH-CH(R)CH₂CO where R=(CH₂)ₙGuanidino (n=1, 2 and 4).

Preferred substitutions include β amino acids β-Ala at position 16, β-Ala or β-hSer at position 17, β-Ala or β-hLeu at position 18, β-Ala or β-hGlu at position 19, and β-Ala at position 20, as shown in the exemplary analogs presented in Table 2. Norleucine (Nle) is preferably substituted for the methionine (Met) at position 18 in those analogs where there is not a β amino acid at position 18.

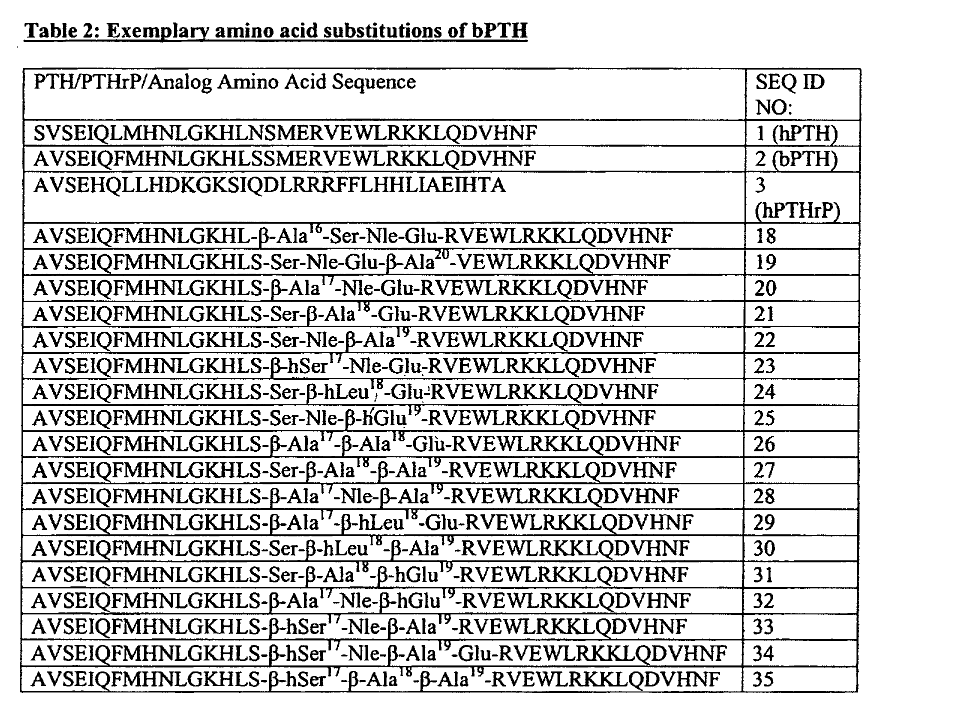

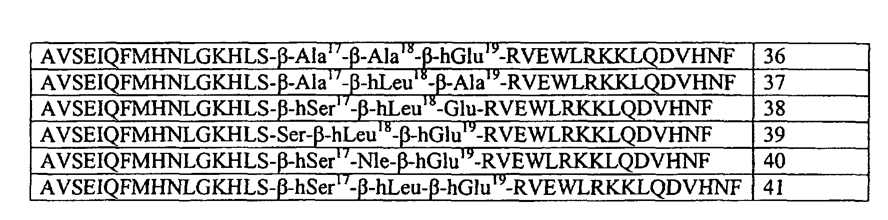

As described in the examples below, one set of preferred analogs of h/bPTH and hPTHrP include the following analogs:

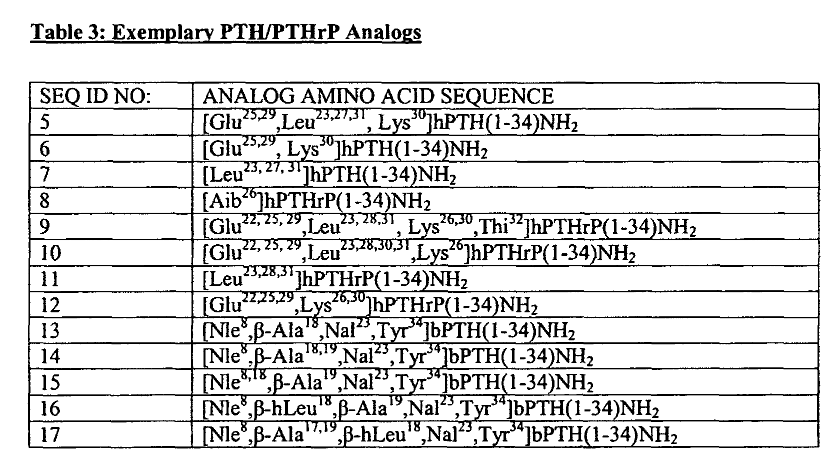

Additional examples of structural permutations in analogs ofh/bPTH and hPTHrP include analogs having one or more pseudopeptide units. Preferred pseudopeptide units include pseudodipeptide units, pseudotripeptide units, etc., which can be located at any series of amino acids in the h/bPTH and hPTHrP analogs. In some preferred embodiments, the pseudodipeptide unit Xxx¹Ψ(CH₂NH)Yyy² can be one of the following: Xxx = Ala, Gly, Thr, Ser and Yyy = Val, Leu, Nle, Ile, Phe. In certain embodiments, analogs containing a pseudodipeptide unit also contain a 2-aminoisobutyric acid residue substitution at position 12 (Aib¹²). Exemplary analogs containing a pseudodipeptide unit include the following:
[Ala¹Ψ(CH₂NH)Val², Nle^{8,18},Aib¹², 2-Nal²³, Tyr³⁴]bPTH(1-34)NH₂ (SEQ ID NO:4)
[Ser¹Ψ(CH₂NH)Val²,Nle^{8,18},Aib¹²,2-Nal²³,Tyr³⁴]hPTH(1-34)NH₂ (SEQ ID NO:42)
[Ser¹Ψ(CH₂NH)Val²,Aib¹²]hPTHrP(1-34)NH₂ (SEQ ID NO:43).

In further embodiments, hPTH, bPTH and hPTHrP analogs contain additional or different amino acid substitutions. For example, instead of the substituted Glu residues mentioned above, one can introduce Asp, or NHCH(R)CO, where R is (CH₂)ₙCO₂H and n=0, 3 and 4. Instead of the substituted Leu residues mentioned above one can introduce Nle, Ile, Val, Ala, Met, or NHCH(R)CO, where R is Et, or nBu. Instead of the substituted Lys residues mentioned above one can introduce Dap, Dbu, or NHCH(R)CO where R is (CH₂)ₙNH₂ and n=5-7.

In still other embodiments, the analogs described herein also can include an amino acid substitution at position 5, preferably a His or Ile substitution. Such substitutions are known to influence binding of PTH and PTHrP to their receptors (see Behar, *et al., Endocrinology* (1996) 137: 4217-4224).

Peptides may be synthesized by any known method in the art including the solid-phase methodology of Merrifield, *J. Am. Chem. Soc.* (1963) 85: 2149-2154 or on an Applied Biosystems 430A peptide synthesizer, using Boc/HOBt/NMP chemistry and *p*-methylbenzhydrylamine·HCl resin. General protocols for the synthesis, purification and characterization of peptides have been reported. See Bisello, *et al., J. Biol. Chem*. (1998) 273: 22498-505 and the PCT application WO 03/105772 for exemplary approaches.

Purity of the synthesized peptide may be determined by analytical RP-HPLC. Structural integrity of the peptides may be confirmed using amino acid analysis and electrospray mass spectrometry.

The biological activity of peptides in terms of IC₅₀ may be measured using a cell assay well known in the art. An exemplary approach using SaOS-2/B10 cells is described in WO 03/105772. Adenylate cyclase activity of PTH peptides also may be determined by methods well known in the art. SaOS-2/B10 cells may be used as described previously. Goldman, *et al., Endocrinology* (1988) 123: 1468. Stably transfected HEK293/C21 also may be used as described by Nakamoto, *et al., Biochemistry* (1995) 34: 10546-10552.

Receptor binding assays and determination of IC50 may be performed using purified receptor or using receptor expressing cells as is well known in the art. For example, IC50 receptor binding for PTH peptides may be determined using HEK293/C21 as described in WO 03/105772.

*In vivo* bone anabolic activities of PTH peptides may be determined by administering the peptide of a formulation containing the peptide into intact animals or an experimental animal model of osteopenia. A rat model of osteoporosis induced by ovariectomy may be used as described previously. See *e.g.* Wronski, *et al., Bone* (1994) 15: 51-58; reviewed in Demster *et al., Endocrine Rev.,* (1993) 14: 690-709.

The bone anabolic effects of the PTH peptide may be determined following 12 to 60 days of treatment by assessing the change in bone mineral density by dual energy x-ray absorption-metry or dry weight of femurs or total ash weight. See *e*.*g*. Hori, *et al., Bone Miner.,* (1988) 3: 193-199. Increase in the rate of bone formation and mineralization may be assessed using metabolic labels, e.g., tetracycline. See Tam, *et al., Endocrinology,* (1982) 110: 506-512. Qualitative and quantitative evaluations of changes in trabecular/cortical bone volume and complexity may be determined by standard histomorphometric methods (see e.g. Podbesek, *et al., Endocrinology* (1983) 112: 1000-1006) of bone samples from control (untreated) and treated animals.

PTH receptor agonists are useful to ameliorate symptoms associated with bone metastasized cancer or bone originated cancer. "Bone metastasized cancer" also known as "secondary" bone cancer or "bone metastases" refers to cancer which has spread from another area of the body to bone. The cells in bone metastases resemble the cells from the cancer's origin. They are not bone cells that have become cancerous, as in the case of primary bone cancers. A wide variety of cancers can cause metastatic bone disease and include carcinomas as well as plasma cell tumor (myeloma) and lymphomas. The more common forms of metastatic bone disease are present in breast cancer, prostate cancer, lung cancer, kidney cancer, thyroid cancer and in myeloma. Carcinomas (cancers that arise from tissues that line or cover organs) are much more likely to metastasize to bone than sarcomas (cancers that originate in connective tissue; cartilage, fat, or muscle). The ribs, pelvis and spine are normally the first bones impacted by bone metastases, while bones more distant from the central skeleton are less frequently affected.

"Bone originated cancer," also known as ''primary bone cancer," is cancer that originates in the bone or tissues ad jointed to the bone such as connective tissue. The most common types of primary bone cancers include osteosarcoma, chondrosarcoma, and Ewing's sarcoma. Osteosarcoma develops in new tissue of growing bones and occurs most commonly in children or adolescents. Chondrosarcoma originates in cartilage, which is a type of connective tissue that serves as a protective layer between bones' ends. Ewing's sarcoma originates in immature nerve tissue within bone marrow. This type of bone cancer also occurs more frequently in children and adolescents. Less common bone cancers include malignant fibrous histocytoma and fibrosarcoma. These cancers are similar to osteosarcoma in that they occur in the extremities, except they occur in adults.

Administration of a pharmaceutical formulation or medicament comprising a PTH receptor agonist to an individual with metastatic cancer to bone is useful for reducing bone loss, reducing the bone fracturing and reducing pain associated with cancer in the bone. The pharmaceutical formulation or medicament may include a single PTH receptor agonist or multiple PTH receptor agonists.

Although many metastatic bone lesions appear overtly lytic, there is usually a local bone formation response that reflects the normal physiology of bone turnover and an attempt to repair bone that is lost. This includes increased osteoblast activity, which can be measured as alkaline phosphatase activity or an increase in uptake of bone scanning agents. However, this repair effort is usually not enough to overcome the lytic activity, the net result being loss of bone with increased pain and other symptoms. Although not wishing to be bound by any mechanism, it is believed that PTH receptor agonists reverse the damage to bone by further increasing the activity of osteoblasts to counteract the increased osteoclast activity.

PTH receptor agonists are administered to patients, including any human patient, in need of such treatment and in an effective amount. An "effective amount" means that amount necessary to delay the onset of, inhibit the progression of, or halt altogether the onset or progression of, the particular condition being treated. Effective amounts will depend on the particular condition being treated, the severity of the condition, individual parameters including age, sex, physical condition, size and weight, concurrent treatment, frequency of treatment, and the mode of administration. These factors are well known to those of ordinary skill in the art and can be addressed with no more than routine experimentation. It is preferred generally that a maximum dose be used, that is, the highest safe dose according to some medical judgment.

As used herein, "reducing bone loss" means that the cancer patient's bone density remains the same, or does not decrease as rapidly as it would without the administration of a PTH receptor agonist. Generally, it is preferable that the patient's bone density remains the same at the time that treatment in initiated and decrease only minimally thereafter during treatment. Preferably, the patient's bone density decrease is inhibited by about 85%, 90%, 95%, 99%, or more (do we want to include the possibility of enhancing bone growth, where the patient's bone density actually increases, in addition to just "reducing bone loss"? or is this not practical or desirable for the application?). When determining the rate of change or amount of change in bone density, patients may serve as their own controls, or their bone densities may be compared to statistically-derived levels, determined, for example, by clinical trials. One such trial is described by Neer, *et al., N. Engl. J. Med*. 344: 1434-41 (2001).

Bone density is easily measured using routine methods. For example, see Neer *et al., supra*, which provides exemplary methods for analysis of the effect of the administration of PTH receptor agonists such as PTH and analogs and/or PTHrP and analogs.

The phrase "reducing bone fracturing" means that the likelihood for fracture is less with treatment than without. The likelihood for fracture may be measured as the delay in time to first fracture following treatment, as compared to no treatment. Preferably, the reduction in bone fracturing is a reduction in pathological bone fracturing as opposed to normal traumatic bone fracturing.

In the context of the present invention, "reducing pain" means that the treatment reduces the level of pain associated with cancer growth in the bone. Reduced pain may occur for sitting positions or for the action of standing up or sitting down. Reduced pain may manifest as a reduction in intensity or duration. A clinically meaningful reduction in pain can be determined by one skilled in the art using any of a variety of standard testing which include a visual analogue scale (VAS), a numerical rating scale from 0 to 10 (NRS), a verbal rating scale (VRS), the Italian Pain Questionnaire (Italian version of the McGill Pain Questionnaire) (PRI), and the Integrated Pain Score (IPS), which is an instrument designed at the Pain Therapy and Palliative Care Division of the National Cancer Institute of Milan to integrate pain intensity and duration in a single measure. De Conno *et al., Pain.* 1994;57(2):161-6. A standard VAS has a scale calibrated zero to 10 on one side with a movable pointer. "no pain" corresponds to zero, and "worst pain imaginable" corresponds to ten. An improvement by at least two levels in the VAS standard scale is considered significant.

Preparations of the invention are administered in pharmaceutically acceptable compositions that may optionally comprise pharmaceutically acceptable salts, buffering agents, preservatives and excipients. Examples of said salts are those of mineral or organic acids, *e*.*g*. of hydrochloric, acetic or methanesulfonic acid. Also salts as alkaline metal or alkaline earth salts, such as sodium or magnesium salts of the carboxylic acid group, are conceivable.

Suitable buffering agents are systems of acetic acid (1-2% w/v), citric acid (1-3% w/v); boric acid (0.5-2.5% w/v), and phosphoric acid (0.8-2% w/v).

Suitable preservatives include benzalkonium chloride (0.003-0.03% w/v); chlorobutanol (0.3-0.9% w/v); parabens (0.01-0.25% w/v) and thimerosal (0.004-0.02% w/v).

Modes of administration selected will *inter alia* depend upon the acuteness and severity of the condition being treated, and the dosage required. Any mode of administration that produces desired therapeutic effect without unacceptable adverse effects is relevant in practicing this invention. Such modes of administration include oral, rectal, topical, transdermal, sublingual or intramuscular, infusion, parenteral, intravenous, intramuscular, intracavity, as an aerosol, buccal, aural, intranasal, inhalation, or subcutaneous. Direct injection could also be preferred for local delivery to the site of injury.

Although at present subcutaneous administration is routinely employed in the administration of most PTH receptor agonists such as PTH and/or PTHrP, oral administration may be preferred for treatment because of the convenience of the patient as well as the dosing schedule. Generally, oral doses of active compounds will be from about 0.1 to 1000 µg per day. It is expected that oral doses in the range of from 0.5 to 50 µg, in one or several administrations per day, will yield the desired results. Intravenous administration would be from an order to several orders of magnitude lower dose per day compared to the oral doses.

Preferably the PTH receptor agonists are administered intermittently, which is known in the art to promote anabolic efficacy of PTH and analogs, PTHrP and analogs. Preferred intermittent administration schedules include daily, every second day, every third day, twice per week, every fourth day, every fifth day, every sixth day, and once per week.

The PTH receptor agonists may conveniently be presented in unit dosage form and may be prepared by any of the methods well known in the art of pharmacy. All methods include the step of bringing the PTH receptor agonists into association with a carrier which constitutes one or more accessory ingredients. In general, the compositions are prepared by uniformly and intimately bringing the PTH receptor agonists into association with a liquid carrier, a finely divided solid carrier, or both, and then, if necessary, shaping the product.

Compositions suitable for parenteral administration conveniently comprise a sterile aqueous preparation of the compounds of the invention preferably isotonic with the blood of the recipient. This aqueous preparation may be formulated according to known methods using those suitable dispersing or wetting agents and suspending agents. The injectable aqueous formulation for Forteo® which contains human PTH(1-34) is exemplary of a suitable pharmaceutical formulation. The preparation may also be a sterile injectable solution or suspension in a diluent or solvent, for example as a solution in 1,3-butane diol. Water, Ringer's solution, and isotonic sodium chloride solution are exemplary acceptable diluents. Sterile, fixed oils may be employed as a solvent or suspending medium. Bland fixed oils, including synthetic mono or di-glycerides, and fatty acids, such as oleic acid, may also be used.

Compositions suitable for oral administration may be presented as capsules, cachets, tablets, syrups, elixirs or lozenges. Compositions suitable for any pulmonary delivery typically are formulated and/or are contained in a nebulizer.

Various approaches for formulating compositions for use in accordance with this invention are described in the HANDBOOK OF PHARMACEUTICAL EXCIPIENTS, 3^{rd} ed., American Pharmaceutical Association, USA and Pharmaceutical Press UK (2000), and PHARMACEUTICS - THE SCIENCE OF DOSAGE FORM DESIGN, Churchill Livingstone (1988).

Pursuant to the present invention, the ameliorating of symptoms that result from growth of bone-metastasizing cancer and bone-originating cancer, by administering a PTH receptor agonist, may be implemented in conjunction with conventional cancer treatments, such as chemotherapy, surgery, and radiation.

The following examples serve to illustrate, and not to limit, the present invention.

### Example 1: Animal model for metastatic bone disease

### a) Model Establishment

An animal model for metastatic bone disease was established in adult C3H mice with murine sarcoma cells as described in Honore, *et al., Neuroscience.* (2000) 98(3):585-98 (see also Clohisy, *et al. J Orthop Res.* (1996) 14(3):396-402). In brief, murine CCL-11 (NCTC clone 2472) osteolytic sarcoma cells were injected into the distal femur. The injection site was closed with dental grade amalgam. Wounds were closed with wound clips. Wound clips were removed at day 6 to prevent interference with behavioral testing. Sham-injected and sarcoma-injected mice received daily subcutaneous injections of either 10 µl/kg vehicle or 10 µl/kg vehicle containing PTH (1-34) at 80 µg/ml concentration beginning after testing and radiographs were taken 6 d after injection of sarcoma cells. Treatment with PTH (1-34) or vehicle ended 14 d after injection, when final behavioral testing was done and the animals sacrificed.

### b) Radiological analysis of bone

To determine bone destruction, radiographs were taken prior to the induction of bone cancer (baseline or BL), and at 6, 10, and 14 days following induction of bone cancer using a Faxitron x-ray machine (Model MX-20). Mice were lightly anesthetized to prevent movement during the x-ray. The radiographs of the mouse femurs were scanned at maximal resolution (2400 dpi) and saved in digital grayscale format (TIFF). The grayscale levels were standardized for all radiographs by setting the amalgam region to white (grayscale value of 255) and the lower limit of the grayscale was set to the darkest pixels of the scanned image. The grayscale values correspond to the luminosity of the scanned images.

The images were analyzed with Metamorph (Universal Imaging Corp, West Chester, PA). The bones were divided into 3 equal regions. Data were taken from the lower region at the site were sarcoma cells were injected. Thresholding was standardized to include all pixels with grayscale values between 2 to 76. Since grayscale values are related to luminosity, an increase in the threshold area represents an increase in darker, or lower density, bone area. The integrated intensity values for the regions were determined with Metamorph. Significant decreases in the intensity of a region indicates a significant reduction in bone density. All measurements for baseline were compared to the baseline control group and all measurements for day 14 were compared to the control group at day 14 to account for expected increases in bone density due to normal growth of the animal.

### c) Behavioral analysis

Spontaneous limb use was assessed as described by Luger et al (2001). The mouse was placed in an empty mouse pan and observed while walking across the pan in a continuous motion. Limping and/or guarding behavior of the right (sarcoma treated) hindlimb was rated on the following scale: 0=complete lack of use, 1=partial non-use, 2=limping and guarding, 3=limping, 4=normal walking.

To assess flinching and guarding behavior, animals were placed in raised plexiglass chambers with a wire grid floor for observation of flinching and guarding of the right hindlimb. The mice were allowed to acclimate to the chamber for 20 minutes. Guarding and flinching behaviors of each mouse were measured for 2 minutes. The number of flinches were counted, and the time spent guarding the foot (the foot is lifted off of the floor) was measured.

Tactile allodynia was determined in the manner described by Chaplan et al. *J Neurosci Methods* (1994) 53(1):55-63. Paw withdrawal threshold was determined in response to probing with calibrated von Frey filaments. The mice were kept in suspended cages with wire mesh floors and the von Frey filament was applied perpendicularly to the plantar surface of the paw of the mouse until it buckled slightly, and was held for 2 sec. A positive response was indicated by a sharp withdrawal of the paw. The 50% paw withdrawal threshold was determined by the non-parametric method of Dixon (Dixon, *Annu Rev Pharmacol Toxicol* 20:441-462, 1980). An initial probe equivalent to 0.4 g was applied and if the response was negative, the stimulus was incrementally increased until a positive response was obtained, then decreased until a negative result was obtained. This up-down method was repeated with 4 more filaments. The pattern of positive and negative responses were tabulated. The 50% paw withdrawal threshold was determined as (10^{[Xf+kδ]})/10,000, where Xf = the value of the last von Frey filament employed, k = Dixon value for the positive/negative pattern, and δ = the mean (log) difference between stimuli. Data were converted to % antiallodynia by the formula: % antiallodynia = 100 x (test value - post-treatment baseline value)/(pretreatment baseline - post-treatment baseline value).

### Example 2: Amelioration of symptoms from metastatic bone disease

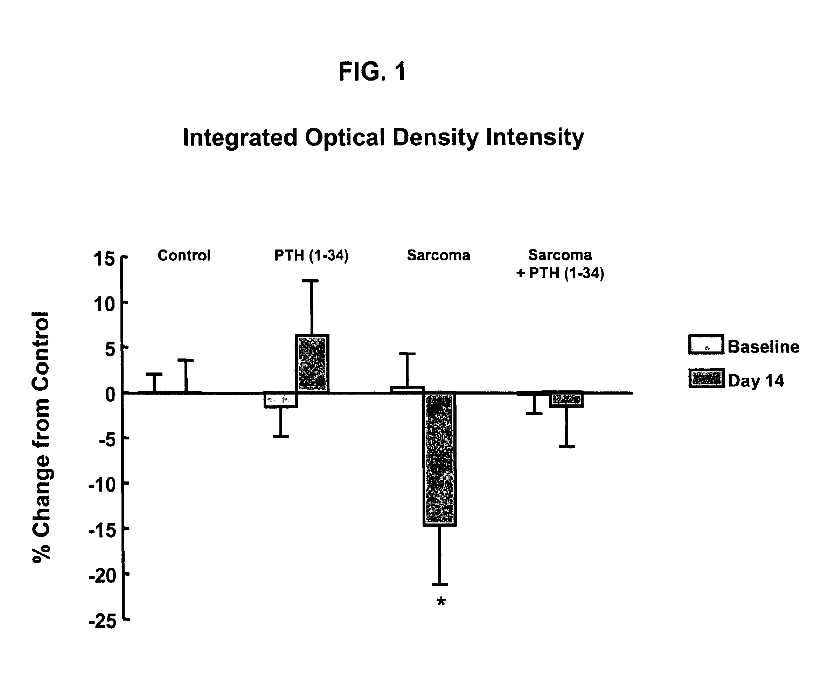

Radiological evaluation of femurs from metastatic bone tumor animals and sham treated animals are shown in FIG. 1. PTH(1-34) treatment of normal animals showed some bone density increase at day 14. Sarcoma bearing animals treated with vehicle showed a prominent decrease in bone density at day 14. In contrast, sarcoma bearing animals treated with PTH showed a near normal bone density at day 14 and an increase in density over that of normal untreated animals at day 17. Thus, these data show that PTH(1-34) reduces bone density loss due to the growth of metastatic cancer in bone.

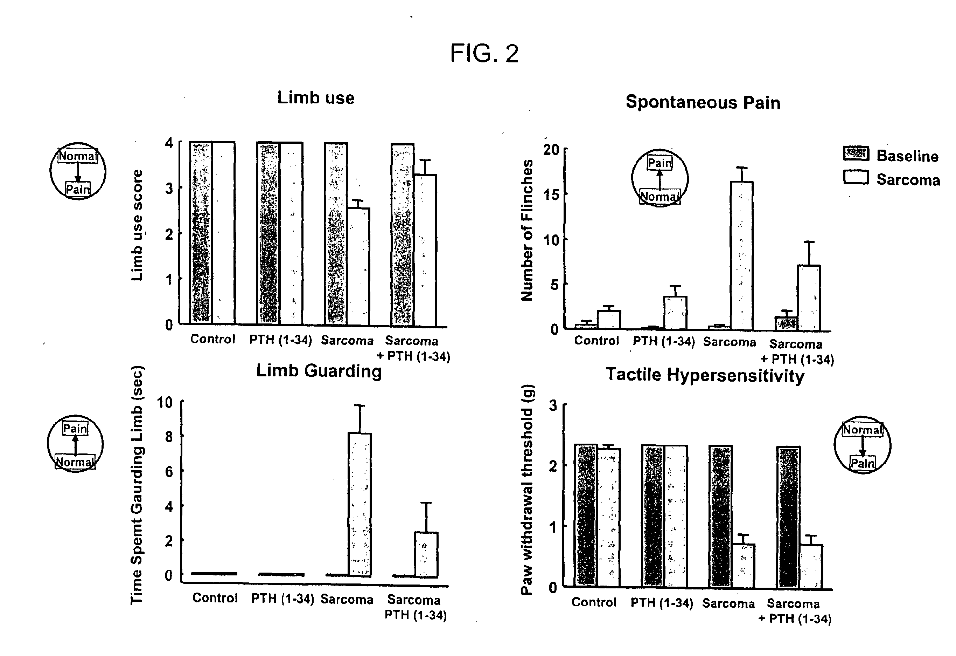

Behavioral effects of PTH treatment in metastatic bone tumor animals are shown in FIG. 2. Limb use decreased in metastatic bone animals returned to near normal levels at day 14 following treatment with PTH (1-34). Limb guarding observed in metastatic bone tumor animals was significantly reduced at day 14 following treatment with PTH(1-34). Similar results were observed for spontaneous pain. Tactile hypersensitivity, reduced in metastatic bone tumor animals was not effected at day 14 following treatment with PTH(1-34). Thus, these data show that PTH(1-34) decreases spontaneous forms of pain, but not evoked pain.

All references cited herein are incorporated in their entirety by reference.

## Claims

1. Use of a PTH receptor agonist for ameliorating symptoms associated with the growth of bone metastasized cancer or bone-originated cancer in the preparation of a medicament which, when administered to an individual in an effective amount reduces bone loss, reduces bone fracturing, or reduces pain.

2. Use according to claim 1, wherein said administered effective amount reduces pain.

3. Use according to any of claims 1-2, wherein said PTH receptor agonist is PTH or an analogue thereof.

4. Use according to claim 3, wherein said PTH or an analogue thereof is PTH (1-34), preferably teriparatide acetate.

5. Use according to any of claims 1-2, wherein said PTH receptor agonist is parathyroid hormone-related protein or an analogue thereof.

6. Use according to any of claims 1-2, wherein said individual has bone metastasized cancer.

7. Use according to claim 6, wherein said bone metastasized cancer is breast cancer, prostate cancer, lung cancer, kidney cancer or thyroid cancer or myeloma.

8. Use according to any of claims 1-2, wherein said individual has bone-originated cancer.

9. Use according to any of claims 1-2, wherein said bone originated cancer is sarcoma.

10. Use according to any one of claims 1-9, wherein the effective amount of the PTH receptor agonist is from 0.1 to 1000 micrograms, preferably from 20 to 200 micrograms.


SEQUENCE LISTING

<110> FERRING B.V.

<120> NOVEL USE

<130> 052209-0113

<150> US 60/538,512
<151> 2004-01-26

<160> 43

<170> PatentIn version 3.2

<210> 1
<211> 34
<212> PRT
<213> Homo sapiens

<400> 1
Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe

<210> 2
<211> 34
<212> PRT
<213> Bos taurus

<400> 2
Ala Val Ser Glu Ile Gln Phe Met His Asn Leu Gly Lys His Leu Ser
1               5                   10                  15

Ser Met Glu Arg Val Glu Trp Leu Arg Lys Lys Leu Gln Asp Val His
            20                  25                  30

Asn Phe

<210> 3
<211> 34
<212> PRT
<213> Homo sapiens

<400> 3
Ala Val Ser Glu His Gln Leu Leu His Asp Lys Gly Lys Ser Ile Gln
1               5                   10                  15

Asp Leu Arg Arg Arg Phe Phe Leu His His Leu Ile Ala Glu Ile His
            20                  25                  30

Thr Ala

<210> 4
<211> 34
<212> PRT
<213> Artificial Sequence

<220>
<223> Synthetic peptide

<220>
<221> MOD_RES
<222> (8)..(8)
<223> Nle

<220>
<221> MOD_RES
<222> (12)..(12)
<223> Aib

<220>
<221> MOD_RES
<222> (18)..(18)
<223> Nle

<220>
<221> MOD_RES
<222> (23)..(23)
<223> 2-Nal

<220>
<223> see specification as filed for preferred embodiments

<400> 4
Ala Val Ser Glu Ile Gln Phe Xaa His Asn Leu Xaa Lys His Leu Ser
1               5                   10                  15

Ser Xaa Glu Arg Val Glu Xaa Leu Arg Lys Lys Leu Gln Asp Val His
            20                  25                  30

Asn Tyr

<210> 5
<211> 34
<212> PRT
<213> Artificial Sequence

<220>
<223> Synthetic peptide

<220>
<223> see specification as filed for preferred embodiments

<400> 5
Ser Val Ser Glu Ile Gln Leu Met His Asn Leu Gly Lys His Leu Asn
1               5                   10                  15

Ser Met Glu Arg Val Glu Leu Leu Glu Lys Leu Leu Glu Lys Leu His
            20                  25                  30

Asn Phe

<210> 6
<211> 34
<212> PRT
<213> Artificial Sequence

<220>
<223> Synthetic peptide

<220>
<223> see specification as filed for preferred embodiments

<400> 6
Ser Val Ser Glu Ile Gln Leu Met His Asn Leu Gly Lys His Leu Asn
1               5                   10                  15

Ser Met Glu Arg Val Glu Trp Leu Glu Lys Lys Leu Glu Lys Val His
            20                  25                  30

Asn Phe

<210> 7
<211> 34
<212> PRT
<213> Artificial Sequence

<220>
<223> Synthetic peptide

<220>
<223> see specification as filed for preferred embodiments

<400> 7
Ser Val Ser Glu Ile Gln Leu Met His Asn Leu Gly Lys His Leu Asn
1               5                   10                  15

Ser Met Glu Arg Val Glu Leu Leu Arg Lys Leu Leu Gln Asp Leu His
            20                  25                  30

Asn Phe

<210> 8
<211> 34
<212> PRT
<213> Artificial Sequence

<220>
<223> Synthetic peptide

<220>
<221> MOD_RES
<222> (26)..(26)
<223> Aib

<220>
<223> see specification as filed for preferred embodiments

<400> 8
Ala Val Ser Glu His Gln Leu Leu His Asp Lys Gly Lys Ser Ile Gln
1               5                   10                  15

Asp Leu Arg Arg Arg Phe Phe Leu His Xaa Leu Ile Ala Glu Ile His
            20                  25                  30

Thr Ala

<210> 9
<211> 34
<212> PRT
<213> Artificial Sequence

<220>
<223> Synthetic peptide

<220>
<221> MOD_RES

<222> (32)..(32)
<223> Thi

<220>
<223> see specification as filed for preferred embodiments

<400> 9
Ala Val Ser Glu His Gln Leu Leu His Asp Lys Gly Lys Ser Ile Gln
1               5                   10                  15

Asp Leu Arg Arg Arg Glu Leu Leu Glu Lys Leu Leu Glu Lys Leu Xaa
            20                  25                  30

Thr Ala

<210> 10
<211> 34
<212> PRT
<213> Artificial Sequence

<220>
<223> Synthetic peptide

<220>
<223> see specification as filed for preferred embodiments

<400> 10
Ala Val Ser Glu His Gln Leu Leu His Asp Lys Gly Lys Ser Ile Gln
1               5                   10                  15

Asp Leu Arg Arg Arg Glu Leu Leu Glu Lys Leu Leu Glu Leu Leu His
            20                  25                  30

Thr Ala

<210> 11
<211> 34
<212> PRT
<213> Artificial Sequence

<220>
<223> Synthetic peptide

<220>
<223> see specification as filed for preferred embodiments

<400> 11
Ala Val Ser Glu His Gln Leu Leu His Asp Lys Gly Lys Ser Ile Gln
1               5                   10                  15

Asp Leu Arg Arg Arg Phe Leu Leu His His Leu Leu Ala Glu Leu His
            20                  25                  30

Thr Ala

<210> 12
<211> 34
<212> PRT
<213> Artificial Sequence

<220>
<223> Synthetic peptide

<220>
<223> see specification as filed for preferred embodiments

<400> 12
Ala Val Ser Glu His Gln Leu Leu His Asp Lys Gly Lys Ser Ile Gln
1               5                   10                  15

Asp Leu Arg Arg Arg Glu Phe Leu Glu Lys Leu Ile Glu Lys Ile His
            20                  25                  30

Thr Ala

<210> 13
<211> 34
<212> PRT
<213> Artificial Sequence

<220>
<223> Synthetic peptide

<220>
<221> MOD_RES
<222> (8)..(8)
<223> Nle

<220>
<221> MOD_RES
<222> (18)..(18)
<223> beta-Ala

<220>
<221> MOD_RES
<222> (23)..(23)
<223> Nal

<220>
<223> see specification as filed for preferred embodiments

<400> 13
Ala Val Ser Glu Ile Gln Phe Xaa His Asn Leu Gly Lys His Leu Ser
1               5                   10                  15

Ser Xaa Glu Arg Val Glu Xaa Leu Arg Lys Lys Leu Gln Asp Val His
            20                  25                  30

Asn Tyr

<210> 14
<211> 34
<212> PRT
<213> Artificial Sequence

<220>
<223> Synthetic peptide

<220>
<221> MOD_RES
<222> (8)..(8)
<223> Nle

<220>
<221> MOD_RES
<222> (18)..(19)
<223> beta-Ala

<220>
<221> MOD_RES
<222> (23)..(23)
<223> Nal

<220>
<223> see specification as filed for preferred embodiments

<400> 14
Ala Val Ser Glu Ile Gln Phe Xaa His Asn Leu Gly Lys His Leu Ser
1               5                   10                  15

Ser Xaa Xaa Arg Val Glu Xaa Leu Arg Lys Lys Leu Gln Asp Val His
            20                  25                  30

Asn Tyr

<210> 15
<211> 34
<212> PRT
<213> Artificial Sequence

<220>
<223> Synthetic peptide


<220>
<221> MOD_RES
<222> (8)..(8)
<223> Nle

<220>
<221> MOD_RES
<222> (18)..(18)
<223> Nle

<220>
<221> MOD_RES
<222> (19)..(19)
<223> beta-Ala

<220>
<221> MOD_RES
<222> (23)..(23)
<223> Nal

<220>
<223> see specification as filed for preferred embodiments

<400> 15
Ala Val Ser Glu Ile Gln Phe Xaa His Asn Leu Gly Lys His Leu Ser
1               5                   10                  15


Ser Xaa Xaa Arg Val Glu Xaa Leu Arg Lys Lys Leu Gln Asp Val His
            20                  25                  30


Asn Tyr



<210> 16
<211> 34
<212> PRT
<213> Artificial Sequence

<220>
<223> Synthetic peptide



<220>
<221> MOD_RES

<222> (8)..(8)
<223> Nle

<220>
<221> MOD_RES
<222> (18)..(18)
<223> beta-hLeu

<220>
<221> MOD_RES
<222> (19)..(19)
<223> beta-Ala

<220>
<221> MOD_RES
<222> (23)..(23)
<223> Nal

<220>
<223> see specification as filed for preferred embodiments

<400> 16
Ala Val Ser Glu Ile Gln Phe Xaa His Asn Leu Gly Lys His Leu Ser
1               5                   10                  15

Ser Xaa Xaa Arg Val Glu Xaa Leu Arg Lys Lys Leu Gln Asp Val His
            20                  25                  30

Asn Tyr

<210> 17
<211> 34
<212> PRT
<213> Artificial Sequence

<220>
<223> Synthetic peptide

<220>
<221> MOD_RES
<222> (8)..(8)
<223> Nle

<220>
<221> MOD_RES
<222> (17)..(17)
<223> beta-Ala

<220>
<221> MOD_RES
<222> (18)..(18)
<223> beta-hLeu

<220>
<221> MOD_RES
<222> (19)..(19)
<223> beta-Ala

<220>
<221> MOD_RES
<222> (23)..(23)
<223> Nal

<220>
<223> see specification as filed for preferred embodiments

<400> 17
Ala Val Ser Glu Ile Gln Phe Xaa His Asn Leu Gly Lys His Leu Ser
1               5                   10                  15

Xaa Xaa Xaa Arg Val Glu Xaa Leu Arg Lys Lys Leu Gln Asp Val His
            20                  25                  30

Asn Tyr

<210> 18
<211> 34
<212> PRT
<213> Artificial Sequence

<220>
<223> Synthetic peptide

<220>
<221> MOD_RES
<222> (16)..(16)
<223> beta-Ala

<220>
<221> MOD_RES
<222> (18)..(18)
<223> Nle

<220>
<223> see specification as filed for preferred embodiments

<400> 18
Ala Val Ser Glu Ile Gln Phe Met His Asn Leu Gly Lys His Leu Xaa
1               5                   10                  15

Ser Xaa Glu Arg Val Glu Trp Leu Arg Lys Lys Leu Gln Asp Val His
            20                  25                  30

Asn Phe

<210> 19
<211> 34
<212> PRT
<213> Artificial Sequence

<220>
<223> Synthetic peptide

<220>
<221> MOD_RES
<222> (18)..(18)
<223> Nle

<220>
<221> MOD_RES
<222> (20)..(20)
<223> beta-Ala

<220>
<223> see specification as filed for preferred embodiments

<400> 19
Ala Val Ser Glu Ile Gln Phe Met His Asn Leu Gly Lys His Leu Ser
1               5                   10                  15

Ser Xaa Glu Xaa Val Glu Trp Leu Arg Lys Lys Leu Gln Asp Val His
            20                  25                  30

Asn Phe

<210> 20
<211> 34
<212> PRT
<213> Artificial Sequence

<220>
<223> Synthetic peptide

<220>
<221> MOD_RES
<222> (17)..(17)
<223> beta-Ala

<220>
<221> MOD_RES

<222> (18)..(18)
<223> Nle

<220>
<223> see specification as filed for preferred embodiments

<400> 20
Ala Val Ser Glu Ile Gln Phe Met His Asn Leu Gly Lys His Leu Ser
1               5                   10                  15

Xaa Xaa Glu Arg Val Glu Trp Leu Arg Lys Lys Leu Gln Asp Val His
            20                  25                  30

Asn Phe

<210> 21
<211> 34
<212> PRT
<213> Artificial Sequence

<220>
<223> Synthetic peptide

<220>
<221> MOD_RES
<222> (18)..(18)
<223> beta-Ala

<220>
<223> see specification as filed for preferred embodiments

<400> 21
Ala Val Ser Glu Ile Gln Phe Met His Asn Leu Gly Lys His Leu Ser
1               5                   10                  15

Ser Xaa Glu Arg Val Glu Trp Leu Arg Lys Lys Leu Gln Asp Val His
            20                  25                  30

Asn Phe

<210> 22
<211> 34
<212> PRT
<213> Artificial Sequence

<220>
<223> Synthetic peptide

<220>
<221> MOD_RES
<222> (18)..(18)
<223> Nle

<220>
<221> MOD_RES
<222> (19)..(19)
<223> beta-Ala

<220>
<223> see specification as filed for preferred embodiments

<400> 22
Ala Val Ser Glu Ile Gln Phe Met His Asn Leu Gly Lys His Leu Ser
1               5                   10                  15

Ser Xaa Xaa Arg Val Glu Trp Leu Arg Lys Lys Leu Gln Asp Val His
            20                  25                  30

Asn Phe

<210> 23
<211> 34
<212> PRT
<213> Artificial Sequence

<220>
<223> Synthetic peptide

<220>
<221> MOD_RES
<222> (17)..(17)
<223> beta-hSer

<220>
<221> MOD_RES
<222> (18)..(18)
<223> Nle

<220>
<223> see specification as filed for preferred embodiments

<400> 23
Ala Val Ser Glu Ile Gln Phe Met His Asn Leu Gly Lys His Leu Ser
1               5                   10                  15

Xaa Xaa Glu Arg Val Glu Trp Leu Arg Lys Lys Leu Gln Asp Val His
            20                  25                  30

Asn Phe

<210> 24
<211> 34
<212> PRT
<213> Artificial Sequence

<220>
<223> Synthetic peptide

<220>
<221> MOD_RES
<222> (18)..(18)
<223> beta-hLeu

<220>
<223> see specification as filed for preferred embodiments

<400> 24
Ala Val Ser Glu Ile Gln Phe Met His Asn Leu Gly Lys His Leu Ser
1               5                   10                  15

Ser Xaa Glu Arg Val Glu Trp Leu Arg Lys Lys Leu Gln Asp Val His
            20                  25                  30

Asn Phe

<210> 25
<211> 34
<212> PRT
<213> Artificial Sequence

<220>
<223> Synthetic peptide

<220>
<221> MOD_RES
<222> (18)..(18)
<223> Nle

<220>
<221> MOD_RES
<222> (19)..(19)
<223> beta-hGlu

<220>
<223> see specification as filed for preferred embodiments

<400> 25
Ala Val Ser Glu Ile Gln Phe Met His Asn Leu Gly Lys His Leu Ser
1               5                   10                  15

Ser Xaa Xaa Arg Val Glu Trp Leu Arg Lys Lys Leu Gln Asp Val His
            20                  25                  30

Asn Phe

<210> 26
<211> 34
<212> PRT
<213> Artificial Sequence

<220>
<223> Synthetic peptide

<220>
<221> MOD_RES
<222> (17)..(17)
<223> beta-Ala

<220>
<221> MOD_RES
<222> (18)..(18)
<223> beta-Ala

<220>
<223> see specification as filed for preferred embodiments

<400> 26
Ala Val Ser Glu Ile Gln Phe Met His Asn Leu Gly Lys His Leu Ser
1               5                   10                  15

Xaa Xaa Glu Arg Val Glu Trp Leu Arg Lys Lys Leu Gln Asp Val His
            20                  25                  30

Asn Phe

<210> 27
<211> 34
<212> PRT
<213> Artificial Sequence

<220>
<223> Synthetic peptide

<220>
<221> MOD_RES
<222> (18)..(19)
<223> beta-Ala

<220>
<223> see specification as filed for preferred embodiments

<400> 27
Ala Val Ser Glu Ile Gln Phe Met His Asn Leu Gly Lys His Leu Ser
1               5                   10                  15

Ser Xaa Xaa Arg Val Glu Trp Leu Arg Lys Lys Leu Gln Asp Val His
            20                  25                  30

Asn Phe

<210> 28
<211> 34
<212> PRT
<213> Artificial Sequence

<220>
<223> Synthetic peptide

<220>
<221> MOD_RES
<222> (17)..(17)
<223> beta-Ala

<220>
<221> MOD_RES
<222> (18)..(18)
<223> Nle

<220>
<221> MOD_RES
<222> (19)..(19)
<223> beta-Ala

<220>
<223> see specification as filed for preferred embodiments

<400> 28
Ala Val Ser Glu Ile Gln Phe Met His Asn Leu Gly Lys His Leu Ser
1               5                   10                  15

Xaa Xaa Xaa Arg Val Glu Trp Leu Arg Lys Lys Leu Gln Asp Val His
            20                  25                  30

Asn Phe

<210> 29
<211> 34
<212> PRT
<213> Artificial Sequence

<220>
<223> Synthetic peptide

<220>
<221> MOD_RES
<222> (17)..(17)
<223> beta-Ala

<220>
<221> MOD_RES
<222> (18)..(18)
<223> beta-hLeu

<220>
<223> see specification as filed for preferred embodiments

<400> 29
Ala Val Ser Glu Ile Gln Phe Met His Asn Leu Gly Lys His Leu Ser
1               5                   10                  15

Xaa Xaa Glu Arg Val Glu Trp Leu Arg Lys Lys Leu Gln Asp Val His
            20                  25                  30

Asn Phe

<210> 30
<211> 34
<212> PRT
<213> Artificial Sequence

<220>
<223> Synthetic peptide

<220>
<221> MOD_RES
<222> (18)..(18)
<223> beta-hLeu

<220>
<221> MOD_RES
<222> (19)..(19)
<223> beta-Ala

<220>
<223> see specification as filed for preferred embodiments

<400> 30
Ala Val Ser Glu Ile Gln Phe Met His Asn Leu Gly Lys His Leu Ser
1               5                   10                  15

Ser Xaa Xaa Arg Val Glu Trp Leu Arg Lys Lys Leu Gln Asp Val His
            20                  25                  30

Asn Phe

<210> 31
<211> 34
<212> PRT
<213> Artificial Sequence

<220>
<223> Synthetic peptide

<220>
<221> MOD_RES
<222> (18)..(18)
<223> beta-Ala

<220>
<221> MOD_RES
<222> (19)..(19)
<223> beta-hGlu

<220>
<223> see specification as filed for preferred embodiments

<400> 31
Ala Val Ser Glu Ile Gln Phe Met His Asn Leu Gly Lys His Leu Ser
1               5                   10                  15

Ser Xaa Xaa Arg Val Glu Trp Leu Arg Lys Lys Leu Gln Asp Val His
            20                  25                  30

Asn Phe

<210> 32
<211> 34
<212> PRT
<213> Artificial Sequence

<220>
<223> Synthetic peptide

<220>
<221> MOD_RES
<222> (17)..(17)
<223> beta-Ala

<220>
<221> MOD_RES
<222> (18)..(18)
<223> Nle

<220>
<221> MOD_RES
<222> (19)..(19)
<223> beta-hGlu

<220>
<223> see specification as filed for preferred embodiments

<400> 32
Ala Val Ser Glu Ile Gln Phe Met His Asn Leu Gly Lys His Leu Ser
1               5                   10                  15

Xaa Xaa Xaa Arg Val Glu Trp Leu Arg Lys Lys Leu Gln Asp Val His
            20                  25                  30

Asn Phe

<210> 33
<211> 34
<212> PRT
<213> Artificial Sequence

<220>
<223> Synthetic peptide

<220>
<221> MOD_RES
<222> (17)..(17)
<223> beta-hSer

<220>
<221> MOD_RES
<222> (18)..(18)
<223> Nle

<220>
<221> MOD_RES

<222> (19)..(19)
<223> beta-Ala

<220>
<223> see specification as filed for preferred embodiments

<400> 33
Ala Val Ser Glu Ile Gln Phe Met His Asn Leu Gly Lys His Leu Ser
1               5                   10                  15

Xaa Xaa Xaa Arg Val Glu Trp Leu Arg Lys Lys Leu Gln Asp Val His
            20                  25                  30

Asn Phe

<210> 34
<211> 35
<212> PRT
<213> Artificial Sequence

<220>
<223> Synthetic peptide

<220>
<221> MOD_RES
<222> (17)..(17)
<223> beta-hSer

<220>
<221> MOD_RES
<222> (18)..(18)
<223> Nle

<220>
<221> MOD_RES
<222> (19)..(19)
<223> beta-Ala

<220>
<223> see specification as filed for preferred embodiments

<400> 34
Ala Val Ser Glu Ile Gln Phe Met His Asn Leu Gly Lys His Leu Ser
1               5                   10                  15

Xaa Xaa Xaa Glu Arg Val Glu Trp Leu Arg Lys Lys Leu Gln Asp Val
            20                  25                  30

His Asn Phe
        35

<210> 35
<211> 34
<212> PRT
<213> Artificial Sequence

<220>
<223> Synthetic peptide


<220>
<221> MOD_RES
<222> (17)..(17)
<223> beta-hSer

<220>
<221> MOD_RES
<222> (18)..(18)
<223> beta-Ala

<220>
<221> MOD_RES
<222> (19)..(19)
<223> beta-Ala

<220>
<223> see specification as filed for preferred embodiments

<400> 35
Ala Val Ser Glu Ile Gln Phe Met His Asn Leu Gly Lys His Leu Ser
1               5                   10                  15


Xaa Xaa Xaa Arg Val Glu Trp Leu Arg Lys Lys Leu Gln Asp Val His
            20                  25                  30


Asn Phe




<210> 36
<211> 34
<212> PRT
<213> Artificial Sequence

<220>
<223> Synthetic peptide


<220>
<221> MOD_RES
<222> (17)..(18)
<223> beta-Ala

<220>
<221> MOD_RES
<222> (19)..(19)
<223> beta-hGlu

<220>
<223> see specification as filed for preferred embodiments

<400> 36
Ala Val Ser Glu Ile Gln Phe Met His Asn Leu Gly Lys His Leu Ser
1               5                   10                  15

Xaa Xaa Xaa Arg Val Glu Trp Leu Arg Lys Lys Leu Gln Asp Val His
            20                  25                  30

Asn Phe

<210> 37
<211> 34
<212> PRT
<213> Artificial Sequence

<220>
<223> Synthetic peptide

<220>
<221> MOD_RES
<222> (17)..(17)
<223> beta-Ala

<220>
<221> MOD_RES
<222> (18)..(18)
<223> beta-hLeu

<220>
<221> MOD_RES
<222> (19)..(19)
<223> beta-Ala

<220>
<223> see specification as filed for preferred embodiments

<400> 37
Ala Val Ser Glu Ile Gln Phe Met His Asn Leu Gly Lys His Leu Ser
1               5                   10                  15

Xaa Xaa Xaa Arg Val Glu Trp Leu Arg Lys Lys Leu Gln Asp Val His
            20                  25                  30

Asn Phe

<210> 38
<211> 34
<212> PRT
<213> Artificial Sequence

<220>
<223> Synthetic peptide

<220>
<221> MOD_RES
<222> (17)..(17)
<223> beta-hSer

<220>
<221> MOD_RES
<222> (18)..(18)
<223> beta-hLeu

<220>
<223> see specification as filed for preferred embodiments

<400> 38
Ala Val Ser Glu Ile Gln Phe Met His Asn Leu Gly Lys His Leu Ser
1               5                   10                  15

Xaa Xaa Glu Arg Val Glu Trp Leu Arg Lys Lys Leu Gln Asp Val His
            20                  25                  30

Asn Phe

<210> 39
<211> 34
<212> PRT
<213> Artificial Sequence

<220>
<223> Synthetic peptide

<220>
<221> MOD_RES
<222> (18)..(18)
<223> beta-hLeu

<220>
<221> MOD_RES
<222> (19)..(19)
<223> beta-hGlu

<220>
<223> see specification as filed for preferred embodiments

<400> 39
Ala Val Ser Glu Ile Gln Phe Met His Asn Leu Gly Lys His Leu Ser
1               5                   10                  15

Ser Xaa Xaa Arg Val Glu Trp Leu Arg Lys Lys Leu Gln Asp Val His
            20                  25                  30

Asn Phe

<210> 40
<211> 34
<212> PRT
<213> Artificial Sequence

<220>
<223> Synthetic peptide

<220>
<221> MOD_RES
<222> (17)..(17)
<223> beta-hSer

<220>
<221> MOD_RES
<222> (18)..(18)
<223> Nle

<220>
<221> MOD_RES
<222> (19)..(19)
<223> beta-hGlu

<220>
<223> see specification as filed for preferred embodiments

<400> 40
Ala Val Ser Glu Ile Gln Phe Met His Asn Leu Gly Lys His Leu Ser
1               5                   10                  15

Xaa Xaa Xaa Arg Val Glu Trp Leu Arg Lys Lys Leu Gln Asp Val His
            20                  25                  30

Asn Phe

<210> 41
<211> 34
<212> PRT
<213> Artificial Sequence

<220>
<223> Synthetic peptide


<220>
<221> MOD_RES
<222> (17)..(17)
<223> beta-hSer

<220>
<221> MOD_RES
<222> (18)..(18)
<223> beta-hLeu

<220>
<221> MOD_RES
<222> (19)..(19)
<223> beta-hGlu

<220>
<223> see specification as filed for preferred embodiments

<400> 41
Ala Val Ser Glu Ile Gln Phe Met His Asn Leu Gly Lys His Leu Ser
1               5                   10                  15


Xaa Xaa Xaa Arg Val Glu Trp Leu Arg Lys Lys Leu Gln Asp Val His
            20                  25                  30


Asn Phe




<210> 42
<211> 34
<212> PRT
<213> Artificial Sequence

<220>
<223> Synthetic peptide


<220>
<221> MOD_RES
<222> (8)..(8)
<223> Nle


<220>
<221> MOD_RES

<222> (12)..(12)
<223> Aib

<220>
<221> MOD_RES
<222> (18)..(18)
<223> Nle

<220>
<221> MOD_RES
<222> (23)..(23)
<223> 2-Nal

<220>
<223> see specification as filed for preferred embodiments

<400> 42
Ser Val Ser Glu Ile Gln Leu Xaa His Asn Leu Xaa Lys His Leu Asn
1               5                   10                  15

Ser Xaa Glu Arg Val Glu Xaa Leu Arg Lys Lys Leu Gln Asp Val His
            20                  25                  30

Asn Tyr

<210> 43
<211> 34
<212> PRT
<213> Artificial Sequence

<220>
<223> Synthetic peptide

<220>
<221> MOD_RES
<222> (12)..(12)
<223> Aib

<220>
<223> see specification as filed for preferred embodiments

<400> 43
Ser Val Ser Glu Ile Gln Leu Met His Asn Leu Xaa Lys His Leu Asn
1               5                   10                  15

Ser Met Glu Arg Val Glu Trp Leu Arg Lys Lys Leu Gln Asp Val His
            20                  25                  30

Asn Phe